# EUROPEAN PATENT SPECIFICATION

(11) **EP 4 021 371 B1**
(45) Date of publication and mention of the grant of the patent: **14.10.2026**
(21) Application number: 20859174.3
(22) Date of filing: 25.08.2020
(51) Int. Cl.: A61J 1/18, A61M 5/168, A61M 5/178, A61M 5/31, A61M 5/315, G16H 20/17, G16H 40/67

(54) **SYRINGE WITH DISPOSABLE BODY AND REUSABLE CAP ENABLING DOSE CAPTURE**
SPRITZE MIT WEGWERFBAREM KÖRPER UND WIEDERVERWENDBARER KAPPE ZUR ERMÖGLICHUNG EINER DOSISAUFNAHME
SERINGUE AVEC CORPS JETABLE ET CAPUCHON RÉUTILISABLE PERMETTANT LA CAPTURE DE DOSE

(30) Priority: 30.08.2019 US 201962894031 P
(43) Date of publication of application: 06.07.2022
(73) Proprietor: Becton, Dickinson and Company, Franklin Lakes, NJ 07417-1880 (US)
(72) Inventor: GYORY, J. Richard, Sudbury, Massachusetts 01776 (US)
(74) Representative: dompatent
(86) International application number: PCT/US2020/047756
(87) International publication number: WO 2021/041385

(56) References cited:
- WO-A1-2017/155672
- WO-A2-2017/070391
- WO-A2-2018/125887
- US-A1- 2010 286 612
- US-A1- 2011 009 821
- US-A1- 2014 018 733
- US-A1- 2016 213 856
- US-A1- 2017 136 185
- US-A1- 2017 286 638
- US-A1- 2017 312 455
- US-A1- 2018 147 362
- US-A1- 2018 333 543
- US-A1- 2019 060 577
- "Vitality Medical: Selecting Syringes and Needles", CANCASTER, 21 April 2015 (2015-04-21), XP055802723, Retrieved from the Internet <URL:https://www.vitalitymedical.com/blog/selecting-syringes-and-needles.html>

## Description

### CROSS-REFERENCE TO RELATED APPLICATION

This Application claims priority from U.S. Provisional Application 62/894,031 filed in the United States Patent and Trademark Office on August 30, 2019.

### BACKGROUND

### 1. Field

Apparatuses and methods consistent with exemplary embodiments relate to syringes for transferring (i.e., injecting or withdrawing) fluids, and more particularly to a syringe including a smart plunger cap that senses and provides medication delivery informatics.

### 2. Description of the Related Art

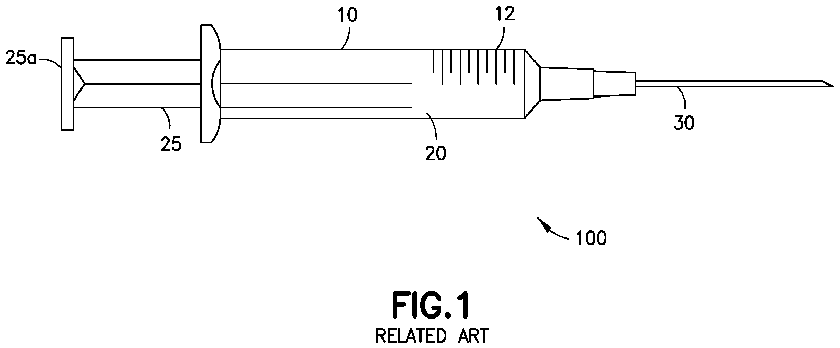

Most syringes in use today are of the disposable or single-use type. A typical disposable syringe 100, illustrated in FIG. 1, is made primarily of plastic and has several key components. The largest, and the one containing the most material, is the plastic barrel 10. The scale printing 12 on the barrel 10 is a critical and costly assembly step that is needed to assure proper dosing by the user. Inside the barrel 10 is a rubber stopper 20 that is used to create a hermetic seal and displace the liquid medication or other fluid into and out of the barrel. A plastic plunger rod 25 interfaces with the rubber stopper 20 to move it back and forth under the user's control. A metal needle 30 or cannula is usually attached to the distal end of the barrel to allow fluids to be injected into or removed from the body, although this is not always the case. For example, a syringe having a male Luer connector at its distal end can be attached to a female Luer connector on a catheter or IV line to inject or withdraw fluids without the use of a needle or cannula.

Large numbers of syringes may be used in a relatively short period of time in hospital and care settings, and for management, by patients, of certain conditions. In the management of diabetes, for example, disposable plastic syringes are often used to administer liquid insulin to a user several times a day. Such single-use syringes typically have clear polymeric barrels with printed scale numbers that a user consults to determine an appropriate dose of insulin from a vial, and fine-gauge metal needles (usually about 6 to 12 mm in length) that inject the dose into the skin with minimal discomfort to the user. The needles may be detachably connected to the barrels using Luer-Lok^{™} or Luer slip connections, or they may be permanently attached or "staked" to the barrels during manufacture of the syringes. Insulin syringes usually have a capacity of 1 ml or less (with 0.3 ml, 0.5 ml and 1.0 ml barrel sizes being common), with scale markings on the barrel representing units of a specific type of insulin (e.g., U-100 or U-500 insulin). Insulin syringes may also be provided with safety features to prevent reuse of the syringe, to shield the used needle, or both. Because insulin syringes are used only once and a user usually requires several of them each day, they are commonly sold in boxes or bags containing multiple syringes.

With respect to insulin syringes of the type described above, there are no durable (reusable) components. The entire syringe and needle are disposed of after a single use, and none of the components are reused. While disposal of a single-use syringe is advantageous in ensuring sterility and preventing the spread of blood-borne diseases, the expense of providing all of the required syringe components and assembly steps for only a one-time use is higher than might be desired. Discarded syringes also create a disposal burden in hospitals and other medical facilities, since they cannot be mixed with other types of medical waste and must instead be placed in dedicated sharps disposal containers. Therefore, a need exists for a syringe in which the expense and disposal burden associated with one-time use is reduced, while preserving the sanitary advantages of a single-use syringe.

Effective administration of some types of drug injections, particularly in the case of insulin used by diabetics, requires that a record be kept of all administered doses. While education is offered for home injection patients, most patients still find it challenging to follow the instructions properly on a daily basis. Additionally, the only means for obtaining a record of injections and dosages injected is by writing it down manually. Health care personnel can record dose-related information in a clinical setting, but there is significant overhead associated with capturing this information. It is also difficult to measure and record certain injection times and dosages. Certain patients may also find it difficult to draw a very specific amount of a drug into a syringe and/or determine a specific amount of a drug that has been injected due to a difficulty in reading scale markings on the barrel of the syringe or in appropriately following instructions.

A need exists for an improved syringe that can provide a user with more accurate information regarding delivered dose and adherence to a prescribed medication dosage regimen.

Documents showing further background art for the present invention are: US 2016/213856 A1, WO 2018/125887 A2, WO 2017/070391 A2, US 2018/333543 A1, US 2019/060577 A1, WO 2017/155672 A1 and US 2017/312455 A1.

### SUMMARY

The present invention is defined in claims 1 and 5.

Exemplary embodiments may address at least the above problems and/or disadvantages and other disadvantages not described above. Also, exemplary embodiments are not required to overcome the disadvantages described above, and may not overcome any of the problems described above.

One or more exemplary embodiments may provide a smart syringe with a disposable body and a reusable smart cap configured to sense and output medication delivery informatics.

According to the invention, a smart syringe is provided and comprises a barrel; a stopper; a plunger rod connected to the stopper such that movement of the plunger rod causes the stopper to be displaced within the barrel; and a smart cap configured to be connected to the plunger rod. The smart cap comprises a sensor configured to sense a movement of the plunger rod, an indicator comprising one of a visual indicator and an audible indicator, a communication module, and a power supply.

The communication module may be a Bluetooth chip.

The plunger rod may comprise a first end connected to the stopper and a second end, opposite the first end, having threading thereon; and the smart cap may be configured to be threaded onto the second end of the plunger rod.

The barrel, the stopper, and the plunger rod may be disposable.

The smart cap further comprises an accelerometer configured to sense when a needle, attached to the barrel, has punctured skin of a user; and a timer configured to measure a time elapsed after the needle has punctured the skin of the user. A microcontroller is configured to control the indicator to output an indication of a predetermined time having passed after the needle has punctured the skin of the user.

According to an another aspect of the invention, a method of obtaining injection information using the syringe according to the invention, is provided, the method comprising powering-on the smart cap by attaching the smart cap to the plunger rod and the barrel of the syringe; sensing, via the sensor disposed in the smart cap, a movement of the plunger rod; calculating a dose administered to a patient using data of movement of the plunger rod output by the sensor; storing data of the dose administered to the patient.

The method may further comprise transmitting the data of the movement of the plunger rod, output by the sensor, from the smart cap to an external device; wherein the calculating the dose and the storing data are performed by the external device.

The method further comprises the microcontroller in the smart cap determining a time of an injection of the dose based on data received from an accelerometer in the smart cap; the timer in the smart cap determining a time elapsed after the time of the injection; and an indicator outputting an indication, to a user, upon a predetermined amount of time having elapsed after the time of the injection.

The method may further comprise obtaining a time of an injection of the dose; and storing data of the time of the injection of the dose.

The obtaining the time of the injection may comprise the accelerometer in the smart cap sensing the injection of the dose, and the microcontroller in the smart cap obtaining the time of the injection based on data output from the accelerometer.

### BRIEF DESCRIPTION OF THE DRAWINGS

The above and/or other aspects will become apparent and more readily appreciated from the following description of example embodiments, taken in conjunction with the accompanying drawings in which:
FIG. 1 illustrates a disposable syringe according to the related art;
FIGs. 2A and 2B illustrate a smart syringe according to an example embodiment;
FIG. 3 illustrates a smart syringe system according to an example embodiment;
FIG. 4 is a schematic diagram of electronic components of a smart cap, according to an example embodiment; and
FIG. 5 is a flowchart of operations according to an example embodiment.

### DETAILED DESCRIPTION

Reference will now be made in detail to example embodiments which are illustrated in the accompanying drawings, wherein like reference numerals refer to like elements throughout. In this regard, the example embodiments may have different forms and may not be construed as being limited to the descriptions set forth herein.

It will be understood that the terms "include," "including", "comprise, and/or "comprising," when used in this specification, specify the presence of stated features, integers, steps, operations, elements, and/or components, but do not preclude the presence or addition of one or more other features, integers, steps, operations, elements, components, and/or groups thereof.

It will be further understood that, although the terms "first," "second," "third," etc., may be used herein to describe various elements, components, regions, layers and/or sections, these elements, components, regions, layers and/or sections may not be limited by these terms. These terms are only used to distinguish one element, component, region, layer or section from another element, component, region, layer or section.

As used herein, the term "and/or" includes any and all combinations of one or more of the associated listed items. Expressions such as "at least one of," when preceding a list of elements, modify the entire list of elements and do not modify the individual elements of the list. In addition, the terms such as "unit," "-er (-or)," and "module" described in the specification refer to an element for performing at least one function or operation, and may be implemented in hardware, software, or the combination of hardware and software.

Various terms are used to refer to particular system components. Different companies may refer to a component by different names - this document does not intend to distinguish between components that differ in name but not function.

Matters of these example embodiments that are obvious to those of ordinary skill in the technical field to which these example embodiments pertain may not be described here in detail.

As discussed above with respect to FIG. 1, a related art disposable syringe 100 includes a plastic barrel 10, having scale printing 12 thereon, and a needle 30 attached thereto. A rubber stopper 20, disposed within the barrel 10, is attached to a plunger rod 25. Pressure on a distal end 25a of the plunger rod 25, puts pressure on a fluid inside the barrel 10, allowing the fluid to be injected into a body.

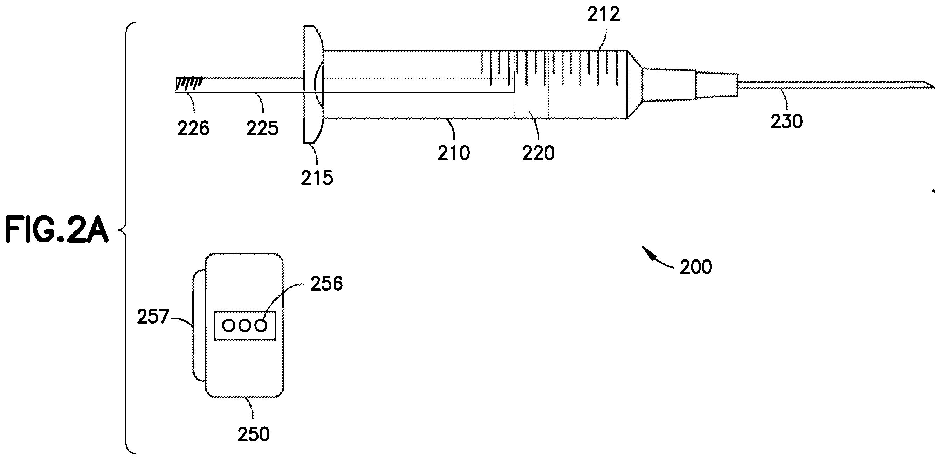

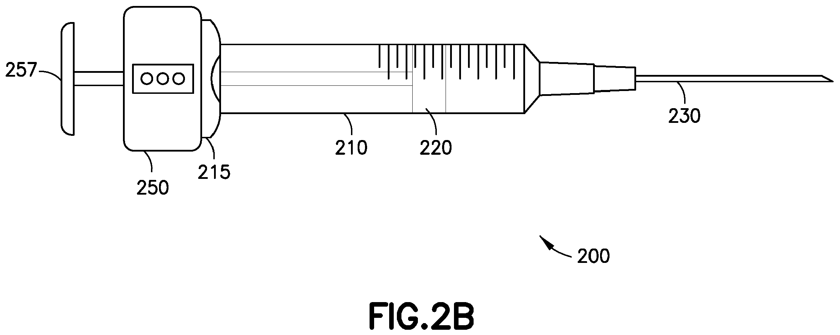

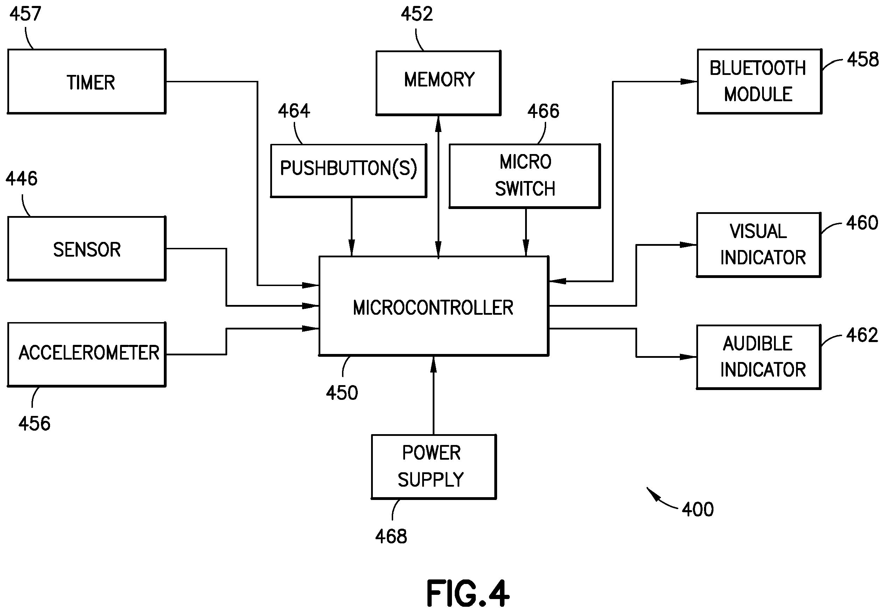

FIGs. 2A and 2B illustrate a smart syringe 200 according to an example embodiment. The smart syringe 200 includes a barrel 210 having a stopper 220 therein and a needle 230 attached or attachable thereto. The needle 230 may be detachably connected to the barrel 210, or may be permanently attached during manufacture. The barrel 210 may have scale printing 212 printed thereon, or the scale printing may be omitted. A plunger rod 225 is attached to the stopper 220. The smart syringe 200 also includes a smart cap 250 and a plunger end cap 257. For purposes of sterility, at least the barrel 210, needle 230, stopper 220, and plunger rod 225 may be disposable. The smart cap 250 and plunger end cap 257 are reusable. For use, the smart cap 250/plunger end cap 257 combination may be threaded onto a threaded end 226 of the plunger rod 225. When assembled together, the plunger rod 225 is engaged with a sensor, described below, within the smart cap 250. The attachment of the smart cap 250 to the barrel 210 and/or the plunger rod 225 may engage a micro switch 466 which allows a supply of power from a power supply 468 to other electronic elements of the smart cap 250, as discussed below with respect to FIG. 4. As discussed in further detail below, the power supply 468 also provides power to a Bluetooth^{™} module which, upon initial powering, can be configured to drive a visual 256/460 and/or audible 462 indicator to indicate a first state.

The smart cap may include a mechanism, such as a dialing mechanism (not illustrated), that allows a dose to be drawn into the syringe from a vial. Such a dialing mechanism may enable the syringe 200 to be converted into a device that behaves and is used in the manner of a pen injector. With such a dialing mechanism, when the plunger is depressed via the end cap 257, the dose is delivered, and the mechanism is reset. Alternatively, in one or more example embodiments, the dialing mechanism may be omitted.

When an injection is complete, the smart cap 250 and end cap 257 may be disconnected from the plunger rod 225 and barrel 210, and the plunger rod 225, barrel 210, stopper 220, and needle 230 may be discarded, while the smart cap 250 and end cap 257 are saved for reuse.

The smart cap 250 may include a sensor 446 to determine the position(s) of the plunger rod 225 during injection. For example, the sensor 446 may convert a linear motion of the plunger rod 225 into a rotary motion of a recording device via a rotary encoder, such as an optical or mechanical rotary encoder, which converts linear motion into degrees of rotation and vice-versa, as would be understood by one of skill in the art.

An accelerometer 456 may also be included to determine when the skin is pierced by the needle 230, enabling a determination of a position of the plunger rod 225 at a time of injection. A timer and an audible 462 and/or visual 256/460 indicator may be included to aid the patient in allowing sufficient time for injection, and/or to provide an indication of one or more states. Typically, a user should not inject a dose and immediately pull the needle from the skin because there is a lag period between injection and when the dose is appropriately placed so that it does not leak back to the surface of the skin. The time of injection may be detected via the accelerometer 456, and a timer 457 may alert the user when it is time to withdraw the needle via the audible 462 and/or visual 256/460 indicator. The visual indicator may comprise one or more lights, such as LEDs, a numeric counter, and/or another type of display screen. The microcontroller may control the visual indicator to display one or more of an amount of a dose, a time of day, a timer, and a number of a dose.

It is clear that a particular dose may be determined by the position(s) of the plunger rod 225 only if the size of the barrel 210 is known. Thus, the smart cap 250 may be configured to attach to only a single size of barrel 210. Alternately, the smart cap 250 and/or a connected device 350 (discussed below) may be informed of the size of the barrel by any of various means, as would be understood by one of skill in the art.

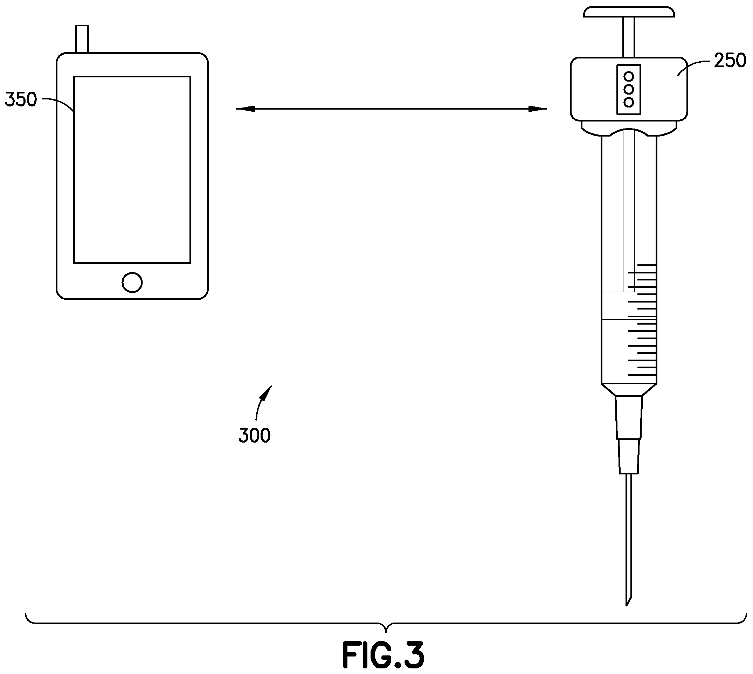

FIG. 3 illustrates a smart syringe system 300 including the smart cap 250 and another connected external device 350. The external device 350 may be, for example, a smart phone, as illustrated, or a laptop, tablet, personal computer, or other processing device. The smart cap 250 and the external device 350 may be connected wirelessly, by Bluetooth, for example, or via a wired connection. The two communicating platforms may have different combinations of hardware and software. Data transfer between the devices may differ depending on when and how data transfer occurs between the smart cap and the external device. For example, the smart cap 250 may transfer data regarding drug delivery status (e.g. complete or incomplete) or other delivery informatics (e.g. rate, timing, etc.) in real time (i.e. during injection) or at any time after injection, such as when previously disconnected devices are eventually paired or otherwise connected. The communication connectivity may be wired or wireless. Different wireless connectivity methods include, but are not limited to Bluetooth^{™}, WiFi, and near field communication (NFC), which may impact device pairing, if needed, and a need for proximity of the devices. The appropriate proximity of the devices relative to each other depends on the connectivity method used, as would be understood by one of skill in the art. The timing of data transfer may depend at least in part on whether or not the two communicating platforms and or at least the smart cap has a time recording capability.

In accordance with an aspect of an example embodiment, the external device may be a smart phone 350 provided with a delivery informatics app to connect to and cooperate with the smart cap 250. A user may pair the smart phone with the smart cap for synchronization using, for example, standard Bluetooth^{™} technology methods.

With continued reference to FIG. 3, data synchronization between the smart cap 250 and the smart phone can occur with every injection, for example, to obtain delivery data. The smart phone 350 advantageously may provide time recording capability (e.g., data provided during or immediately after an injection is stored at a memory device in the smart phone 350 with a time stamp using a clock in the smart phone). Bluetooth^{™} connectivity between the smart phone 350 and the delivery device allows the smart cap 250 to be within about 10 meters of the smart phone 350 and operable to transfer delivery data to the smart phone. The pairing with a smart phone 350 for data transfer and use of the smart phone's memory and time recording features allow for electronic components in the smart cap 250 to be minimized for reduced complexity and reduced cost of manufacture.

FIG.4 is a schematic diagram of the electronic components 400 within the smart cap 250. The smart cap 250 may also perform other condition monitoring and information reporting functions, according to one or more example embodiments. The components of the smart cap 250 include a microcontroller 450 with an internal time-of-day clock, a sensor 446, a memory 452 for storing programming and data used by the microcontroller 450, an accelerometer 456 for measuring the amount of motion or perturbation to which the plunger rod 225 may be subjected, a communication module 458 for communicating with the connected device, and a micro switch 466 that senses an initial connection of the plunger rod 225 to the smart cap 250 and of the smart cap 250 to the barrel of the syringe 100. The communication module may comprise a wired connector (e.g. a USB or mini USB interface) or a wireless interface to communicate, for example, via Bluetooth^{™} or WiFi or NFC technology. For example, the smart cap 250 may include a Bluetooth^{™} chip, for example a Bluetooth^{™} low energy LE chip such as TI CC 2541 which has an on-board processor and memory for synchronization and other Bluetooth^{™} operations.

The smart cap 250 may additionally include one or more visual indicators 256/460 such as differently colored light emitting diodes (LEDs), one or more audible and/or tactile indicators 462 such as beepers, buzzers, speakers or vibrating devices, and one or more pushbuttons 464. A power supply 468, for example in the form of a replaceable or rechargeable direct current (DC) battery and suitable voltage regulating circuitry, supplies power to the microcontroller 450 and to any of the other components of FIG. 4 that require electrical power.

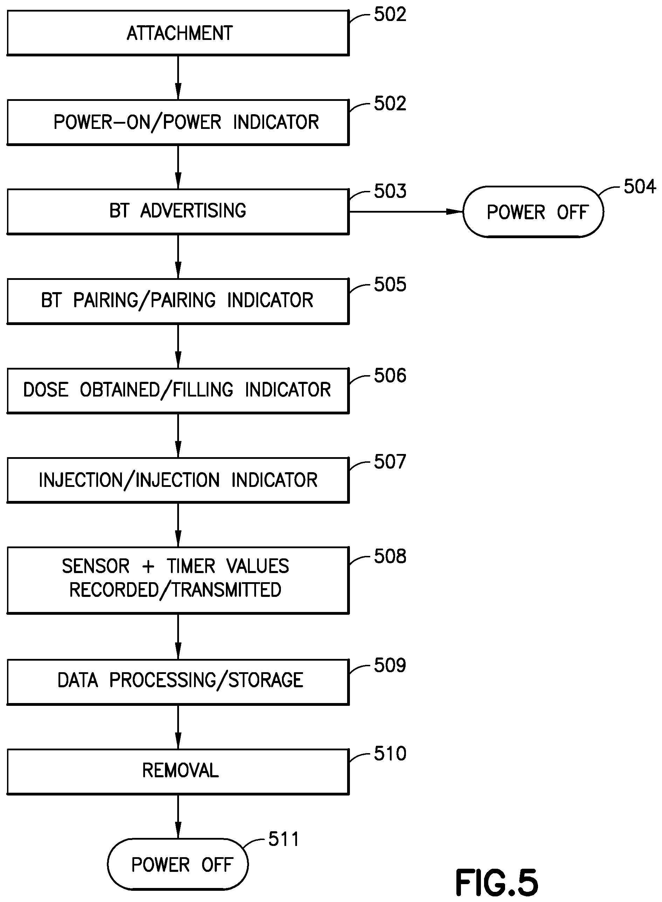

FIG. 5 is a flow chart of operations performed by a user and by the smart cap according to an example embodiment. As discussed above, an attachment of the smart cap 250 to the plunger rod 225 and barrel 210 (block 501) causes the micro switch 466 to close and power from the power supply 468 to be supplied to the smart cap 250. One or both of the visual indicator 256/460 and audible indicator 462 may indicate that the smart cap 250 is powered-on, but not yet connected to an external device (block 502). The microcontroller 450 is configured such that the powering-on of the smart cap 250 powers the Bluetooth^{™} or other connection module 458 to commence advertising to pair with the external device 350 (block 503). If no pairing occurs, the smart cap 250 is powered off (block 504).

If pairing between the connection module 48 of the smart cap 250 and the external device 350 is successful, one or both of the visual indicator 256/460 and audible indicator 462 may indicate that the devices are connected (block 505). When the user pulls back the plunger rod 225 in order to obtain a dose, i.e. filling the barrel 210 of the syringe 200, the sensor 446 senses this movement of the plunger rod 225, and the indicator(s) 256/460 and/or 462 provide a filling indication to the user (block 506). The user can then inject the dose, and upon detection by the sensor 446 and the accelerometer 456 of injection of the dose, the indicator(s) may provide an injection indication to the user (block 507). The timer 457 may also transmit a time of injection to the microcontroller 450, and the sensor 446 may sense the movement of the plunger rod 225, during injection, and transmit this information to the microcontroller 450. Information from the timer 457 and/or sensor may be recorded in the memory 452 and transmitted to the external device 350 (block 508). During and/or after injection, the external device 350 can be configured by the app to process received data to determine, for example, a time of the injection, a flow rate over time, and a total dose and to store that information (block 509). Either the microcontroller 450 of the smart cap 250 or the external device 350 may determine a time and volume of a dose, and a dose rate over time, for example, can be stored in the external device 350. After the injection is completed, the user separates the smart cap 250 from the plunger rod 225 and barrel 210 of the syringe (block 510), and the smart cap 250 is powered off (block 511).

With further reference to the indicator(s) 256/460 and 462, the visual indicator 256/460 may include one or more LEDs to show one or more states. The audible indicator may output a sound, such as a tone or a pre-recorded voice, for example, to indicate the one or more states. For example, one or both of the indicator(s) 256/460 and 462 may indicate one or more of the following states: (1) the smart cap 250 is powered and advertising (e.g., both operations can happen at the same time and, if a time limit expires, the device may be powered off); (2) the smart cap 250 is paired with an external device 350; (3) insulin or another medication is being pulled into the syringe 200; (4) an injection is in progress; and/or (5) the user may remove the needle from the injection site. This last indication may provide an additional benefit to the user. As mentioned above, typical syringe users are instructed to deliver a prescribed dose and then count to 10, which provides only a very subjective and likely erroneous delivery indication. The smart cap 250, by contrast, is configured to sense when the injection is in progress, and to operate a countdown timer that alerts the user when it is safe to remove the needle from the injection site. A single LED or a single tone can be used to indicate multiple states, such as all five of the previously mentioned states. For example, an RGB LED can indicate different colors that may correspond to device states, and an LED may flash and/or a tone may sound in different patterns as well depending on injection status.

As discussed above, according to an aspect of an example embodiment, data may be stored in the memory 452 of the smart cap 250, as well as transmitted to an external device 350. Thus, data can be stored in the smart cap 250 and transmitted to the external device 350 at a later time than during real-time injection and sensing operations. Thus, data is not lost if the smart cap 250 and the external device 350 are not paired at the time of data capture.

It may be understood that the exemplary embodiments described herein may be considered in a descriptive sense only and not for purposes of limitation. Descriptions of features or aspects within each exemplary embodiment may be considered as available for other similar features or aspects in other exemplary embodiments.

While exemplary embodiments have been described with reference to the figures, it will be understood by those of ordinary skill in the art that various changes in form and details may be made therein without departing from the scope as defined by the following claims.

## Claims

1. A smart syringe (200), comprising:
a barrel (210);
a stopper (220);
a plunger rod (225) connected to the stopper (220) such that movement of the plunger rod (225) causes the stopper (220) to be displaced within the barrel (210); and
a smart cap (250) configured to be connected to the plunger rod (225), the smart cap (250) comprising:
a sensor (446) configured to sense a movement of the plunger rod (225),
an indicator (256, 462) comprising one of a visual indicator (256) and an audible indicator (462),
a communication module (458), and
a power supply (468),
**characterized in that**
the smart cap (250) further comprises an accelerometer (456) configured to sense when a needle (230), attached to the barrel (210), has punctured skin of a user, wherein:
the smart cap (250) further comprises a timer (457) configured to measure a time elapsed after the needle (230) has punctured the skin of the user; and
a microcontroller (450) is configured to control the indicator (256, 462) to output an indication of a predetermined time having passed after the needle (230) has punctured the skin of the user.

2. The smart syringe (200) of claim 1, wherein the communication module (458) comprises a Bluetooth chip.

3. The smart syringe (200) of claim 1, wherein:
the plunger rod (225) comprises a first end connected to the stopper (220) and a second end, opposite the first end, having threading thereon; and
the smart cap (250) is configured to be threaded onto the second end of the plunger rod (225).

4. The smart syringe (200) of claim 1, wherein the barrel (210), the stopper (220), and the plunger rod (225) are disposable.

5. A method of obtaining injection information using the syringe (200) of any one of claims 1-4, the method comprising:
powering on the smart cap (250) by attaching the smart cap (250) to the plunger rod (225) and the barrel (210) of the syringe (200);
sensing, via the sensor (446) disposed in the smart cap (250), a movement of the plunger rod (225);
calculating a dose administered to a patient using data of movement of the plunger rod output by the sensor (446); and
storing data of the dose administered to the patient,
**characterized in that** further comprising:
the microcontroller (450) in the smart cap (250) determining a time of an injection of the dose based on data received from the accelerometer (456) in the smart cap (250);
the timer (457) in the smart cap (250) determining a time elapsed after the time of the injection; and
the indicator (256, 462) outputting an indication, to a user, upon a predetermined amount of time having elapsed after the time of the injection.

6. The method of claim 5, further comprising:
transmitting the data of the movement of the plunger rod (225), output by the sensor (446), from the smart cap (250) to an external device (350);
wherein the calculating the dose and the storing data are performed by the external device (350).

7. The method of claim 5, further comprising:
obtaining a time of an injection of the dose; and
storing data of the time of the injection of the dose.

8. The method of claim 5, wherein the obtaining the time of the injection comprises the accelerometer (456) in the smart cap (250) sensing the injection of the dose, and the microcontroller (450) in the smart cap (250) obtaining the time of the injection based on data output from the accelerometer (456).

## Patentansprüche

1. Intelligente Spritze (200), die aufweist:
einen Zylinder (210);
einen Stopfen (220);
eine Kolbenstange (225), die derart mit dem Stopfen (220) verbunden ist, dass eine Bewegung der Kolbenstange (225) bewirkt, dass der Stopfen (220) innerhalb des Zylinders (210) verlagert wird; und
eine intelligente Kappe (250), die dazu ausgebildet ist, mit der Kolbenstange (225) verbunden zu werden, wobei die intelligente Kappe (250) aufweist:
einen Sensor (446), der dazu ausgebildet ist, eine Bewegung der Kolbenstange (225) zu erfassen,
einen Indikator (256, 462), der entweder einen visuellen Indikator (256) oder einen akustischen Indikator (462) umfasst,
ein Kommunikationsmodul (458) und
eine Stromversorgung (468),
**dadurch gekennzeichnet, dass**
die intelligente Kappe (250) ferner einen Beschleunigungsmesser (456) aufweist, der dazu ausgebildet ist, zu erfassen, wann eine am Zylinder (210) angebrachte Nadel (230) die Haut eines Benutzers durchstochen hat,
wobei:
die intelligente Kappe (250) ferner einen Timer (457) aufweist, der dazu ausgebildet ist, eine Zeit zu messen, die verstrichen ist, nachdem die Nadel (230) die Haut des Benutzers durchstochen hat; und
ein Mikrocontroller (450) dazu ausgebildet ist, den Indikator (256, 462) derart anzusteuern, dass dieser einen Hinweis darauf ausgibt, dass eine vorbestimmte Zeit verstrichen ist, nachdem die Nadel (230) die Haut des Benutzers durchstochen hat.

2. Intelligente Spritze (200) nach Anspruch 1, wobei das Kommunikationsmodul (458) einen Bluetooth-Chip aufweist.

3. Intelligente Spritze (200) nach Anspruch 1, wobei:
die Kolbenstange (225) ein erstes Ende, das mit dem Stopfen (220) verbunden ist, und ein dem ersten Ende gegenüberliegendes zweites Ende aufweist, das mit einem Gewinde versehen ist; und
die intelligente Kappe (250) dazu ausgebildet ist, auf das zweite Ende der Kolbenstange (225) aufgeschraubt zu werden.

4. Intelligente Spritze (200) nach Anspruch 1, wobei der Zylinder (210), der Stopfen (220) und die Kolbenstange (225) wegwerfbar sind.

5. Verfahren zum Erhalten von Injektionsinformationen unter Verwendung der Spritze (200) nach einem der Ansprüche 1 bis 4, wobei das Verfahren umfasst:
Einschalten der intelligenten Kappe (250) durch Anbringen der intelligenten Kappe (250) an der Kolbenstange (225) und dem Zylinder (210) der Spritze (200);
Erfassen einer Bewegung der Kolbenstange (225) mittels des in der intelligenten Kappe (250) angeordneten Sensors (446);
Berechnen einer einem Patienten verabreichten Dosis unter Verwendung von Daten über die Bewegung der Kolbenstange, die von dem Sensor (446) ausgegeben werden; und
Speichern von Daten über die dem Patienten verabreichte Dosis,
**dadurch gekennzeichnet, dass** das Verfahren ferner umfasst:
Bestimmen eines Zeitpunkts einer Injektion der Dosis durch den Mikrocontroller (450) in der intelligenten Kappe (250) auf Grundlage von Daten, die von dem Beschleunigungsmesser (456) in der intelligenten Kappe (250) empfangen werden;
Bestimmen einer seit dem Zeitpunkt der Injektion verstrichenen Zeit durch den Timer (457) in der intelligenten Kappe (250); und
Ausgeben eines Hinweises an einen Benutzer durch den Indikator (256, 462), sobald nach dem Zeitpunkt der Injektion eine vorbestimmte Zeitdauer verstrichen ist.

6. Verfahren nach Anspruch 5, ferner umfassend:
Übertragen der vom Sensor (446) ausgegebenen Daten über die Bewegung der Kolbenstange (225) von der intelligenten Kappe (250) an eine externe Vorrichtung (350);
wobei das Berechnen der Dosis und das Speichern der Daten von der externen Vorrichtung (350) durchgeführt werden.

7. Verfahren nach Anspruch 5, ferner umfassend:
Ermitteln eines Zeitpunkts einer Injektion der Dosis; und
Speichern von Daten über den Zeitpunkt der Injektion der Dosis.

8. Verfahren nach Anspruch 5, wobei das Ermitteln des Zeitpunkts der Injektion umfasst, dass der Beschleunigungsmesser (456) in der intelligenten Kappe (250) die Injektion der Dosis erfasst und der Mikrocontroller (450) in der intelligenten Kappe (250) den Zeitpunkt der Injektion auf Grundlage von Daten ermittelt, die von dem Beschleunigungsmesser (456) ausgegeben werden.

## Revendications

1. Seringue intelligente (200), comprenant :
un cylindre (210) ;
un bouchon (220) ;
une tige de piston (225) reliée au bouchon (220) de sorte que le mouvement de la tige de piston (225) entraîne le déplacement du bouchon (220) à l'intérieur du cylindre (210) ; et
un capuchon intelligent (250) configuré pour être relié à la tige de piston (225), le capuchon intelligent (250) comprenant :
un capteur (446) configuré pour détecter un mouvement de la tige de piston (225),
un indicateur (256, 462) comprenant l'un d'un indicateur visuel (256) et d'un indicateur sonore (462),
un module de communication (458), et
une alimentation électrique (468),
**caractérisée en ce que**
le capuchon intelligent (250) comprend en outre un accéléromètre (456) configuré pour détecter le moment où une aiguille (230), fixée au cylindre (210), a perforé la peau d'un utilisateur,
dans laquelle :
le capuchon intelligent (250) comprend en outre un temporisateur (457) configuré pour mesurer le temps écoulé après que l'aiguille (230) a perforé la peau de l'utilisateur ; et
un microcontrôleur (450) est configuré pour commander l'indicateur (256, 462) afin qu'il délivre en sortie une indication qu'un temps prédéterminé s'est écoulé après que l'aiguille (230) a perforé la peau de l'utilisateur.

2. Seringue intelligente (200) selon la revendication 1, dans laquelle le module de communication (458) comprend une puce Bluetooth.

3. Seringue intelligente (200) selon la revendication 1, dans laquelle :
la tige de piston (225) comprend une première extrémité reliée au bouchon (220) et une seconde extrémité, opposée à la première extrémité, sur laquelle se trouve un filetage ; et
le capuchon intelligent (250) est configuré pour être vissé sur la seconde extrémité de la tige de piston (225).

4. Seringue intelligente (200) selon la revendication 1, dans laquelle le cylindre (210), le bouchon (220) et la tige de piston (225) sont jetables.

5. Procédé d'obtention d'informations d'injection en utilisant la seringue (200) selon l'une quelconque des revendications 1 à 4, le procédé comprenant :
la mise sous tension du capuchon intelligent (250) en fixant le capuchon intelligent (250) à la tige de piston (225) et au cylindre (210) de la seringue (200) ;
la détection, via le capteur (446) disposé dans le capuchon intelligent (250), d'un mouvement de la tige de piston (225) ;
le calcul d'une dose administrée à un patient en utilisant des données relatives au mouvement de la tige de piston délivrées en sortie par le capteur (446) ; et
le stockage des données relatives à la dose administrée au patient,
**caractérisé en ce qu'**il comprend en outre :
la détermination, par le microcontrôleur (450) dans le capuchon intelligent (250), du moment d'une injection de la dose sur la base des données reçues de l'accéléromètre (456) dans le capuchon intelligent (250) ;
la détermination, par le temporisateur (457) dans le capuchon intelligent (250), du temps écoulé depuis le moment de l'injection ; et
la sortie, par l'indicateur (256, 462), d'une indication à un utilisateur lorsqu'une quantité de temps prédéterminée s'est écoulée depuis le moment de l'injection.

6. Procédé selon la revendication 5, comprenant en outre :
la transmission des données relatives au mouvement de la tige de piston (225), délivrées en sortie par le capteur (446), depuis le capuchon intelligent (250) vers un dispositif externe (350) ;
dans lequel le calcul de la dose et le stockage des données sont effectués par le dispositif externe (350).

7. Procédé selon la revendication 5, comprenant en outre :
l'obtention d'un moment d'injection de la dose ; et
le stockage des données relatives au moment de l'injection de la dose.

8. Procédé selon la revendication 5, dans lequel l'obtention du moment de l'injection comprend la détection, par l'accéléromètre (456) dans le capuchon intelligent (250), de l'injection de la dose, et l'obtention, par le microcontrôleur (450) dans le capuchon intelligent (250), du moment de l'injection sur la base des données délivrées en sortie par l'accéléromètre (456).
